(19) Europäisches Patentamt
European Patent Office
Office européen des brevets

(11) Publication number: 0 003 732
B1

(12) EUROPEAN PATENT SPECIFICATION

(45) Date of publication of patent specification: 19.10.83

(51) Int. Cl.$^3$: C 07 D 233/56, C 07 D 233/58, A 61 K 31/415

(21) Application number: 78101294.3

(22) Date of filing: 02.11.78

# (54) Imidazole derivatives and salts thereof, their synthesis, and pharmaceutical formulations thereof.

(30) Priority: 01.02.78 GB 398478

(43) Date of publication of application: 05.09.79 Bulletin 79/18

(45) Publication of the grant of the patent: 19.10.83 Bulletin 83/42

(84) Designated Contracting States: BE CH DE FR LU NL SE

(56) References cited: US - A - 3 927 017

(73) Proprietor: THE WELLCOME FOUNDATION LIMITED
183-193 Euston Road
London NW1 2BP (GB)

(72) Inventor: Thorogood, Peter Brian
114, Venner Road
Sydenham London, S.E.26 (GB)

(74) Representative: Berg, Wilhelm, Dr. et al,
Dr. Berg, Dipl.-Ing. Stapf, Dipl.-Ing. Schwabe, Dr. Dr. Sandmair Patentanwälte Postfach 860245
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 003 732 B1

Imidazole derivatives and salts thereof, their synthesis and pharmaceutical formulations thereof

The present invention relates to imidazole derivatives and salts thereof, to their synthesis and intermediates therefor, to pharmaceutical formulations containing such compounds and to the use of these compounds in medicine.

Thromboxane $A_2$ ($TXA_2$), a potent stimulator of blood platelet aggregation, is produced, in platelets, from the prostaglandin endoperoxides $PGG_2$ and $PGH_2$. Prostacyclin ($PGI_2$), which has potent anti-aggregatory activity, is also produced (in blood vessel walls) from $PGG_2$ and $PGH_2$ and it has been suggested that a balance between the production of $TXA_2$ and $PGI_2$ is the controlling factor in thrombus formation. It would, in consequence be desirable in the treatment and prophylaxis of thrombo-embolic disorders to be able to selectively inhibit $TXA_2$ synthetase, thereby favouring the production of the anti-aggregatory agent $PGI_2$.

Imidazole and 1-methylimidazole are known to provide some degree of inhibition of the enzymic conversion of the endoperoxides ($PGG_2$ and $PGH_2$) to thromboxane $A_2$ by platelet microsomes (Moncada *et al.,* Prostaglandins, *13*/4, 611—618 1977). Certain 1-*n*-alkylimidazoles, especially 1-*n*-dodecylimidazole and its higher homologues have been described as being capable of lowering serum cholesterol levels (U.K. Patent No. 1 364 312; Biochem. Pharmacol. *24*, 1902—1903, 1975).

We have now discovered that $TXA_2$ synthetase may be inhibited by 1-arylalkylimidazoles of formula (I) and acid addition salts thereof. The compounds of formula (I) and their salts are hereinafter referred to as the "active compounds".

The compounds of formula (I) are novel and of formula:

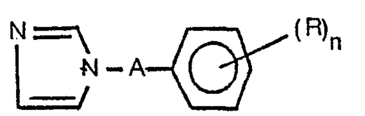 (I)

in which A is a straight or branched alkylene group of from 1 to 3 carbon atoms, or a straight or branched alkenylene or alkynylene group of 2 or 3 carbon atoms, n is an integer which is at least 1, and the or each R substituent, which when n is greater than 1 may be the same of different, is a saturated hydrocarbon group of from 1 to 4 carbon atoms, or an unsaturated hydrocarbon group of from 2 to 4 carbon atoms with the provisos that

(a) when A is a methylene or ethylidine group, n is at least 2 when R is a saturated hydrocarbon group,

(b) when A is a branched propylene, or straight propylidene group, n is at least 3 when R is a saturated hydrocarbon group,

(c) when A is unsaturated R may also be selected from alkoxy of from 1 to 4 carbon atoms; alkylenedioxy of from 1 to 4 carbon atoms, when n is at least 2; halo; trihalomethyl; hydroxy; carboxyl; a salt of such a carboxyl group; carboalkoxy; carboaryloxy; carboarylalkyloxy; $—NR^6R^7$ or $—CONR^6R^7$, in which $R^6$ and $R^7$ may be the same or different and are hydrogen or alkyl of from 1 to 4 carbon atoms; with the further proviso that when n is 1, R is not a saturated hydrocarbon group;

or an acid addition salt of such a 1-arylalkylimidazole. It should be noted that various 1-(mono-substituted-benzyl)-imidazoles are described, for example, in U.K. patent specification 1148103 and in J. Med. Chem., 1975, 18(8), 833—36 in a non-pharmaceutical context. Furthermore, various compounds of general formula (I) wherein A is a branched propylene group, R is a saturated hydrocarbon group and n is 1 or 2 are described for example in U.S. patent specifications Nos. 4115578 and 3769422, also in a non-pharmaceutical context.

In formula (I) examples of the group A are:—

methylene,
propylene, and
in the orientation of formula (I),
$—CH_2—CH{=}CH$, (*cis* or *trans* or isomeric mixture thereof).

A valuable class of compounds of formula (I) are those in which the aromatic ring is substituted by at least two saturated or unsaturated alkyl radicals, especially if one substituent is in the 4-position in the benzene ring and A is either methylene ($—CH_2—$) or, in the orientation of formula (I), $—CH_2—CH{=}CH—$ (*cis* or *trans* or a *cis/trans* mixture-cinnamyl compounds). When A is unsaturated, preferred compounds are those in which the aromatic ring contains alkyl, chloro or methoxy substituents.

Compounds of formula (I) may also be used as acid addition salts thereof, especially as pharmaceutically acceptable ones.

Especially preferred compounds include:—

1-(3,4-dimethylbenzyl)imidazole
1-(2,4-dichlorocinnamyl)imidazole i.e. 1-[3-(2,4-dichlorophenyl)prop-2-enyl]imidazole
1-[3-(2,6-dichlorophenyl)prop-2-enyl]imidazole, and acid addition salts thereof.

Other preferred compounds include:—

1-(2,4,6-trimethylbenzyl)imidazole
1-[3-(3,4,5-trimethoxyphenyl)prop-2-enyl]imidazole
1-[3-(3,4-dimethoxyphenyl)prop-2-enyl]imidazole
1-[3-(2-hydroxyphenyl)prop-2-enyl]imidazole
1-[3-(3-bromophenyl)prop-2-enyl]imidazole
1-[3-(4-chlorophenyl)prop-2-enyl]imidazole
1-[3-(3,4-dimethylphenyl)prop-2-enyl]imidazole
1-[3-(2-methoxyphenyl)prop-2-enyl]imidazole, and acid addition salts thereof.

In contrast to imidazole and 1-methylimidazole, the compounds of formula (I) are more potent inhibitors of $TXA_2$ synthetase. Many of the compounds (for example in formula (I) R is 3,4-dimethyl and A is $—CH_2—$ or in the orientation of formula (I), $—CH_2CH{=}CH—$) are also more selective in their action in not inhibiting other anti-aggregatory prostaglandin-generating enzymes. The compounds of formula (I) also do not produce the side-effects found with imidazole upon *in vivo* administration. The compounds of formula (I) are further capable of inhibiting platelet aggregation *in vivo* and also are capable of disaggregating platelet clumps, the compounds 1-(3,4-dimethylbenzyl)imidazole, 1-(2,4-dichlorocinnamyl)imidazole and 1-(2,6-dichlorocinnamyl)imidazole, and their salts especially displaying these properties.

Imidazoles of formula (I) and acid addition salts thereof may be made by any method known in the art for the synthesis of compounds of analogous structure. In general these methods comprise linking the imidazole ring to the remainder of the molecule; converting a precursor molecule by elimination of a functional group; and formation of the desired compound form a corresponding pyrazole, imidazoline or other unsaturated analogue.

A most convenient method of synthesis involves the reaction of imidazole formula (II) or a salt thereof with an arylalkylating agent of formula (III):

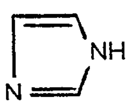

(II)

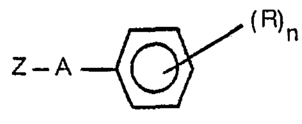

(III)

wherein R, n, and A are as defined in formula (I) and Z is a leaving group. This reaction is well established in the literature, and the leaving group may be chosen from a variety of substituents but especially halo, preferably chloro or bromo, or from p-toluenesulphonyloxy but other arylsulphonyloxy, alkanesulphonyloxy or arylalkylsulphonyloxy radicals may be used. The reaction is preferably performed in the presence of an acid acceptor, for example an alkali metal alkoxide, such as sodium methoxide or potassium tertiary butoxide, in the presence of an alkanol. The leaving group Z may itself be formed *in situ* from the corresponding alkanol (Z=OH) by reaction with a hydrohalogenic acid (e.g. hydrochloric acid or a Lewis acid, such as aluminium chloride: see Japanese Patent Kokai No. 131577/77) and the resulting agent of formula (III) reacted directly with imidazole without prior isolation. Alternatively an alkanol (Z=OH) or a derivative thereof (e.g.

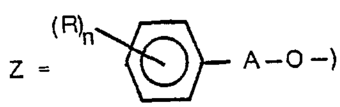

may be reacted directly with imidazole (II) by heating in the presence of a dehydrating agent, such as phosphoric acid, or a phosphate (see Japanese Patent Publication No. 51 105 060), sulphuric acid or sulphates (see Japanese Patent Publication No. 51 105 061).

Among precursor molecules which may be converted to a compound of formula (I) or an acid addition salt thereof, are substituted imidazole derivatives of formula (IV) or addition salts thereof

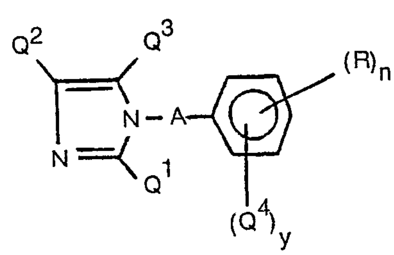

(IV)

wherein A, n and R are as defined in formula (I), and $Q^1$, $Q^2$, $Q^3$ and $Q^4$ are the same or different, at least one being a radical capable of removal by, for example reduction or oxidation, the remaining radical or radicals being selected from hydrogen or a radical capable of removal in the same or another manner (e.g. a carboxyl group — see formula (VI) — removed by decarboxylation), y is 0 or an integer with the proviso that y and n together do not exceed 5. $Q^1$, $Q^2$, $Q^3$ and $Q^4$ may be selected for example from thio (—SH), alkylthio (—S-alkyl, wherein alkyl has from 1 to 4 carbon atoms) or halo preferably chloro or bromo. The reaction conditions are chosen according to the nature of the radicals $Q^1$, $Q^2$, $Q^3$ and $Q^4$. Desulphurisation may be performed by oxidative or reductive procedures using for example nitric acid or Raney nickel; and reductive dehalogenation by the use of zinc and acetic acid or Raney nickel or other reagents known in the art or described in the literature.

Another class of examples include carboxyimidazoles or derivatives thereof of formula (VI);

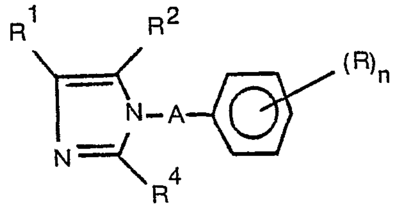

(VI)

wherein A, n, and R are as defined in formula (I), at least one of $R^1$, $R^2$ and $R^4$ is carboxyl or a derivative thereof (for example an ester such as an alkyl ester, an acid halide such as the chloride, or the nitrile) and the other(s) is hydrogen or carboxyl or a derivative as described. The compounds of formula (VI) may be converted into the imidazoles of formula (I) by any suitable decarboxylation conditions which may simply comprise heating the compounds with or without a catalyst, such as copper.

The imidazoles of formula (I) may also be made from a compound of formula (VII):

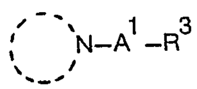

(VII)

wherein

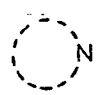

is 1-imidazoline, 1-imidazole or 1-pyrazole, $A^1$ is straight or branched saturated or unsaturated acyclic, hydrocarbon radical which may include a keto-group, and $R^3$ is

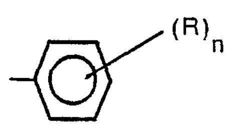

wherein R and n are as defined in formula (I) and when A is unsaturated R may also be nitro provided that at least one of

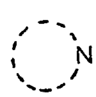

$A^1$ and $R^3$ is other than 1-imidazole, a saturated acyclic hydrocarbon group and

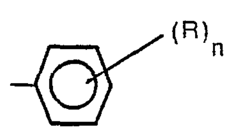

as defined in formula (I). Thus an imidazoline (VIII):

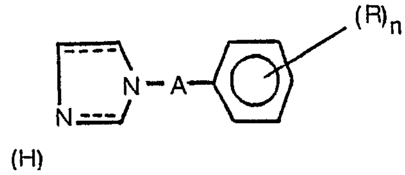

(VIII)

wherein one of ------ represents an extra bond, and A, n and R are as defined in formula (I), may be dehydrogenated to the corresponding imidazole in the presence of a catalyst, for example by heating to 250°C in the presence of palladium, nickel or platinum under pressure, or by heating with a dehydrogenating agent, such as selenium or copper oxide 1-Pyrazole compounds (VII) may be treated with ultra-violet irradiation, optionally under an inert atmosphere (e.g. argon) in for example 1,2-dimethoxyethane at room or elevated temperatures (see for example "Ring Transformations of Heterocycles" edited van der Plas, Academic Press, 1973 at page 261). The unsaturated imidazoles of formula (I) (in formula (VII), $A^1$ and/or R (within $R^3$) are unsaturated) may be reduced to corresponding less unsaturated or completely saturated compounds (but not reducing the aromatic nucleus) by hydrogenation with a noble metal catalyst for example platinum or palladium in an alkanol. If R is amino in the final product then its precursor may be a nitrogen-containing group reducible to amino e.g. nitro. A compound for example of formula

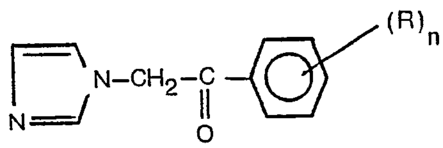

(IX)

where R and n are as for formula (I), may be reduced at the keto group to a $—CH_2—$ group for example by a Clemmensen reduction. Where one or more of the R groups is a saturated or unsaturated alkyl group it may be introduced into the phenyl ring by a Friedel Crafts or similar Lewis-acid catalysed reaction of the type.

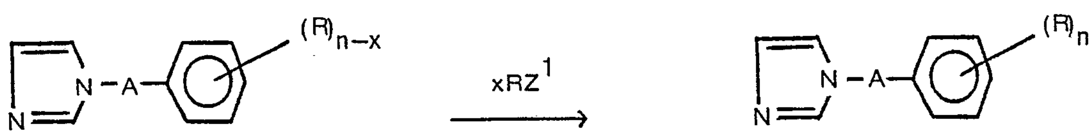


(X) (I)

wherein A, R and n are as defined for formula (I), x is an integer less than or equal to n and $Z^1$ is a leaving group, e.g. halo, suitable for use in this type of alkylation.

Compounds of formula (I) may also be prepared by cyclising preferably in the presence of an acid acceptor, a compound of formula

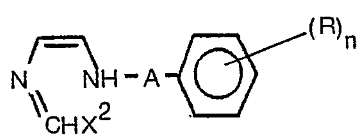

(XI)

wherein A, R and n are as defined for formula (I) and $X^2$ is a leaving group.

Compounds of formula (I) may also be prepared by reacting a compound of formula

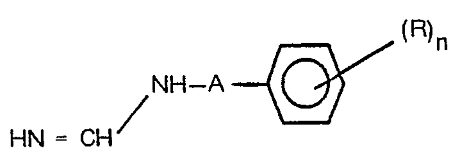

(XII)

wherein A, R and n are as defined for formula (I), with a compound of formula:

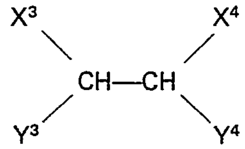

(XIII)

wherein either of $X^3$ and $Y^3$ is a leaving group such as halo or hydroxy and the other is hydrogen, or $X^3$ and $Y^3$ are both halo or together form a keto group or an acetal derivative thereof e.g. both $X^3$ and $Y^3$ are alkoxy, and $X^4$ and $Y^4$ are as defined for $X^3$ and $Y^3$, although they may be the same as or different from $X^3$ and $Y^3$.

An imine salt of for example formula

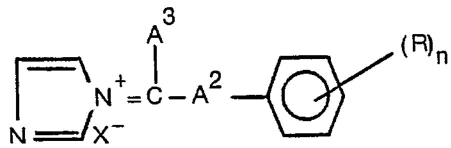

(XIIIa)

(wherein R and n are as for formula I, $X^-$ is an anion, $A^2$ is a chemical bond or a straight or branching saturated or unsaturated acyclic hydrocarbon radical, which may include a keto group, $A^3$ is hydrogen or a saturated or unsaturated acyclic hydrocarbon radical, which may include a keto group, with the proviso that $A^2$ and $A^3$ together contain no more than 2 carbon atoms) may be reduced to the corresponding compound of formula (I), by e.g. zinc and a mineral acid, e.g. hydrochloric acid.

The intermediates for use in the above described reactions may also be made by conventional methods known in the art. Thus the 1-pyrazole and 1-imidazoline intermediates (formula VII) may be prepared by alkylation of pyrazole and imidazoline in an analogous manner to that described above for preparation of the corresponding imidazoles. The intermediates of formula (III) may be made in known manner preferably by halogenation of the corresponding alcohols (formula (III), Z = — OH) where A is unsaturated in such compounds the alcohol is conveniently prepared from paraformaldehyde and a precursor molecule with an unsaturated A group containing two carbon atoms (cf Bull. Chem. Soc. Japan, 46/8, 2512—5, 1973). The substituted imidazole intermediates of formula (IV) may be made in known manner, for example see "Imidazole and its derivatives" Part I, Ed. K. Hofmann, Interscience Publishers Inc. New York, 1973. For example the 2-thioimidazoles of formula (IV) may be made by cyclisation of an acetal of formula (XIV):

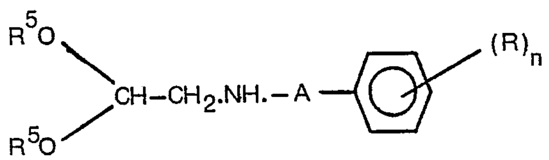

(XIV)

with thiocyanate, wherein $R^5$ is alkyl, aryl or arylalkyl.

The pharmaceutically acceptable addition salts of the compounds of formula (I) may be prepared by any method known in the art. In particular they may be prepared by treating the parent imidazole with the appropriate acid.

Examples of the addition salts of the compounds of formula (I) include those salts derived from the following acids: oxalic, hydrochloric, hydrobromic, sulphuric, nitric, perchloric, fumaric, maleic, phosphoric, glycollic, lactic, salicylic, succinic, toluene-p-sulphonic, tartaric, acetic, citric, methanesulphonic, formic, benzoic, malonic, naphthalene-2-sulphonic and benzenesulphonic.

The imidazoles of formula (I) may be used in conjunction with a phosphodiesterase inhibitor, which provides a further synergistic increase in effect, as it acts against platelet aggregation by a different pathway.

Suitable phosphodiesterase inhibitors for use in potentiating the anti-aggregatory effects of the active compounds include as such or as pharmaceutically acceptable salts:—

(a) Xanthine derivatives such as:—

Theophylline(3,7-dihydro-1,3-dimethyl-1*H*-purine-2,6-dione), and salts thereof;
3-Isobutyl-1-methyl-xanthine;
Coffeine(3,7-dihydro-1,3,7-trimethyl-1*H*-purine-2,6-dione) and salts thereof, and
Aminophylline (adduct of Theophylline and 1,2-ethanediamine (2:1)).

(b) Isoquinoline derivatives, for example:—
Papaverine (6,7-Dimethoxy-1-(3,4-dimethoxybenzyl)-iso-quinoline) and salts thereof; and
6,7-Diethoxy-1-(4,5-diethoxybenzyl)isoquinoline or its salts e.g. its hydrochloride.

(c) Derivatives of pyrimido(5,4-d-pyrimidine, for example:—
Dipyridamole(2,2′,2‴-(4,8-dipiperidino-pyrimido[5,4-d]pyrimidin-2,6-diyldinitrilo-tetraethanol) and its salts;
2,2′,2‴-[[4-(1-piperidinyl)pyrimido[5,4-d]pyrimidin-2,6-diyl]dinitrilo]tetrakisethanol and its salts; and
2,4,6-tri-4-morpholinylpyrimido[5,4-d]pyrimidine and its salts.

(d) Derivatives of thieno[3,2-d]pyrimidine, for example:—
N-[4-(4-morpholinyl)thieno[3,2-d]pyrimidin-2-yl]-1,2-ethanediamine.

(e) Derivatives of pyrazolo[3′,4′:2,3]pyrido[4,5-b][1,5]benzodiazepin-6-(3*H*)-one, for example:—
3-Ethyl-7,12-dihydro-7,12-dimethylpyrazolo[4′,3′:5,6]pyrido[4,3-b]1,5-benzodiazepin-6(3*H*)-one;
10-Chloro-3-ethyl-7,12-dimethyl-7,12-dihydropyrazolo[4′,3′:5,6]pyrido[4,3-b][1,5]benzo-diazepin-6-(3*H*)-one.

(f) Derivatives of 1*H*- or 2*H*-pyrazolo[3,4-b]pyridine, for example:—
4-(Butylamino)-1-ethyl-1*H*-pyrazolo[3,4-b]pyridine-5-carboxylic acid ethyl ester.
4-(Butylamino)-1*H*-pyrazolo[3,4-b]pyridine-6-carboxylic acid ethyl ester;
4-Chloro-1-ethyl-3-methyl-1*H*-pyrazolo[3,4-b]pyridine-5-acetonitrile;
1-Ethyl-4-(isopropylidenehydrazino)-3-methyl-1*H*-pyrazolo[3,4-b]pyridine-5-carboxylic acid ethyl ester or its salts such as its hydrochloride hemihydrate; and
2-Methyl-6-phenyl-4-(1-piperidinyl)-2*H*-pyrazolo[3,4-b]pyridine or its salts e.g. its hydrochloride.

(g) Derivatives of 5*H*-furo[3,4-d]pyrazolo[3,4-b]pyridine-5-one, for example:—
4-(Butylamino)-1-ethyl-1,7-dihydro-7-hydroxy-5*H*-furo[3,4-e]pyrazolo[3,4-b]pyridin-5-one; and

(h) Derivatives of 1-(2*H*)-naphthalenone, for example:—
2-[(Dimethylamino)methyl]-3,4-dihydro-7-methoxy-1(2*H*)-naphthalenone or its salts e.g. its 1:1 hydrochloride.

The active compounds are particularly useful in the treatment and/or prophylaxis of thrombo-embolic disorders in mammals, including man. It is to be understood that the term "thromboembolic disorders" includes those disorders whose etiology is associated with platelet aggregation.

The active compounds are useful wherever it is desired to inhibit platelet aggregation and/or to reduce the adhesive character of platelets, and consequently to treat or prevent the formation of thrombi in mammals, including man. For example, the compounds are useful in the treatment and prevention of myocardial infarcts, cerebro-vascular thrombosis and ischaemic peripheral vascular disease; to treat and prevent post-operative thrombosis; and to promote patency of vascular grafts following surgery.

The active compounds are also useful as an addition to blood, blood products, blood substitutes, and other fluids which are used in artificial extra-corporeal circulation and perfusion of isolated body portions, e.g., limbs and organs, whether attached to the original body, detached and being preserved or prepared for transplant, or attached to a new body. It may also be used in laboratory animals, e.g. cats, dogs, rabbits, monkeys and rats, for these purposes in order to develop new methods and techniques for organ and limb transplants.

The active compounds also exhibit some vasodilatory action on blood vessels and therefore have a utility as anti-hypertensives for the treatment of high blood pressure in mammals, including man.

The amount of active compound required for therapeutic or prophylactic effect will vary with the route of administration, and the nature of the condition under treatment. In general a suitable dose for a mammal, including man, of active compound will lie in the range of 0.1 to 300 mg per kg body weight, particularly from 0.5 to 10 mg per kg body weight, for example 2 mg per kg. A suitable single oral dose for an adult human lies within the range of 50 to 600 mg, for example 150 mg given say three times a day.

While it is possible for the active compounds to be administrered as the raw chemical it is preferable to present them as a pharmaceutical formulation. The formulations, both for veterinary and for human medical use, of the present invention comprise an active compound as above defined, together with one or more acceptable carriers therefor and optionally other therapeutic ingredients. The carrier(s) must be "acceptable" in the sense of being compatible with the other ingredients of the

formulation and not deleterious to the recipient thereof. Unit doses of a formulation may contain between 60 mg and 1.5 g of an active compound.

The formulations include those suitable for oral, rectal, vaginal or parenteral (including subcutaneous, intramuscular and intravenous) administration. Preferred formulations include tablets, capsules and injectable suspensions or solutions.

The formulations may conveniently be presented in unit dosage form and may be prepared by any of the methods well known in the art of pharmacy. All methods include the step of bringing into association the active compound (in the form of the base or a pharmaceutically acceptable acid addition salt) with the carrier which constitutes one or more accessory ingredients. In general the formulations are prepared by uniformly and intimately bringing into association the active compound with liquid carriers or finely divided solid carriers or both, and then, if necessary, shaping the product into the desired formulation.

It will be appreciated from the foregoing that the present invention provides the following features:—

(a) Novel 1-arylalkylimidazoles of formula (I), and acid addition salts thereof.

(b) Methods of preparing imidazoles of formula (I) and acid addition salts thereof.

(c) Pharmaceutical formulations containing the imidazoles of formula (I) or a pharmaceutically acceptable acid addition salt thereof and a pharmaceutically acceptable carrier.

(d) Method of preparing the pharmaceutical formulations containing the imidazoles of formula (I) or a pharmaceutically acceptable acid addition salt thereof.

(f) A 1-arylalkylimidazole of formula (I) or salt thereof as an active agent for the treatment of a thrombo-embolic disorder in a mammal or mammalian tissue, including man or human tissue.

The following Examples are provided by way of an illustration of the present invention and should in no way be construed as constituting a limitation thereof. All temperatures are given in degrees Celsius.

## Example 1

Preparation of 1-(3,4-Dimethylbenzyl)imidazole

1-Chloromethyl-3,4-dimethylbenzene (34.76 g, 0.225 mol) was added to a mixture of imidazole (13.6 g, 0.2 mol) and sodium bicarbonate (16.8 g, 0.2 mol) in dry methanol (100 ml). Following the addition, the reaction mixture was stirred and heated under reflux for 3 h.

After cooling, the reaction mixture was filtered, and the filtrate was evaporated under reduced pressure to afford a yellow oil. The residue was extracted with chloroform (3 × 100 ml), and the combined extracts were washed with saturated brine (100 ml). The chloroform solution was dried over magnesium sulphate, and then concentrated under reduced pressure. The resulting oil was purified using a silica gel column and ethyl acetate/methanol (9:1) as eluent. The product fractions were pooled, concentrated, and the resulting oil was distilled to afford 1-(3,4-dimethylbenzyl)imidazole b.p. 128—130°/0.3 mm Hg.

## Example 2

Salts of 1-(3,4-Dimethylbenzyl)imidazole

A. Hydrogen Fumarate

A solution of fumaric acid (0.29 g, 0.0025 mol) in hot ethanol (10 ml) was added to a stirred solution of 1-(3,4-dimethylbenzyl)imidazole (0.46 g, 0.0025 mol) in hot ethanol (10 ml). After boiling for 0.25 h, the solution was evaporated to afford a white solid. Recrystallisation of the solid from ethyl acetate afforded 1-(3,4-dimethylbenzyl)imidazole hydrogen fumarate 1/6 hydrate as a white solid m.p. 138—140°.

B. Hydrogen Succinate

A hot solution of succinic acid (0.295 g, 0.0025 mol) in hot ethanol (20 ml) was added to a stirred, hot solution of 1-(3,4-dimethylbenzyl)imidazole (0.46 g, 0.0025 mol) in hot ethanol (10 ml). After boiling for 0.25 h, the solution was evaporated under reduced pressure to afford a white solid. Recrystallisation of the solid from ethyl acetate/petroleum ether (b.p. 40—60°) afforded 1-(3,4-dimethylbenzyl)imidazole hydrogen succinate as white crystals, m.p. 134—135°.

C. Hydrogen Oxalate

A hot solution of oxalic acid (0.225 g, 0.0025 mol) in dry ethanol (10 ml) was added to a solution of 1-(3,4-dimethylbenzyl)imidazole (0.46 g, 0.0025 mol) in hot ethanol (20 ml). After boiling for 0.25 h, the solution was evaporated to afford a white solid. Recrystallisation of the solid from ethanol/petroleum ether (b.p. 40—60°) afforded 1-(3,4-dimethylbenzyl)imidazole hydrogen oxalate as a white solid, m.p. 92—93°.

## Example 3

Preparation of 1-[3-(2,4-Dichlorophenyl)prop-2-enyl]imidazole

1-Chloro-3-(2,4-dichlorophenyl)prop-2-ene (11.1 g, 0.05 mol) was added dropwise to a stirred

solution of imidazole (3.4 g, 0.05 mol) and potassium *tert*-butoxide (5.6 g, 0.05 mol) in butan-1-ol (100 ml). Following the addition, the reaction mixture was stirred and heated under reflux for 3.5 h.

After cooling, the reaction mixture was filtered, and the filtrate was concentrated under reduced pressure. Hydrochloric acid (150 ml 2M) was then added to the residue and the aqueous mixture was washed with ether (1 × 60 ml). The acidic solution was then basified with sodium hydroxide solution (10 M) and the resulting oil was extracted with chloroform. The chloroform extracts were combined and dried over magnesium sulphate. Evaporation of the chloroform under reduced pressure afforded a pale yellow oil which was purified using a silica gel column and by elution with ethyl acetate/methanol (9:1). The product fractions were pooled and concentrated to afford an oil which was distilled, to afford 1 - [3 - (2,4 - dichlorophenyl)prop - 2 - enyl]imidazole, b.p. 144—148°/0.007 mmHg.

Example 4

Preparation of 1-[3-(2,6-Dichlorophenyl)prop-2-enyl]imidazole

1-Chloro-3-(2,6-Dichlorophenyl)prop-2-ene (11.1 g 0.05 mol) was added dropwise to a stirred solution of imidazole (3.4 g, 0.05 mol) and potassium *tert*-butoxide (5.6 g, 0.05 mol) in butan-1-ol (100 ml). Following the addition, the reaction mixture was stirred and heated under reflux for 3.5 h.

After cooling, the reaction mixture was filtered, and the filtrate was concentrated under reduced pressure. Hydrochloric acid (150 ml, 2M) was then added to the residue, and the aqueous mixture was washed with ether (1 × 60 ml). The acidic solution was then basified with sodium hydroxide solution (10 M), and the resulting oil was extracted with chloroform. The chloroform extracts were combined and dried over magnesium sulphate. Evaporation of the chloroform under reduced pressure afforded a pale yellow oil which was purified using a silica gel column and by elution with ethyl acetate/methanol (9:1). The product fractions were pooled and concentrated to afford an oil which was distilled, to afford 1 - [3 - (2,6 - dichlorophenyl)prop - 2 - enyl]imidazole, b.p. 156—158°/0.02 mm Hg.

Example 5

Biological Results

Horse platelets were prepared from whole horse blood by differential centrifugation. Approximately $10^6$ platelets were homogenised in 1 ml 100 mM Tris buffer pH 7.4. Various concentrations of active compound were added and the reaction sets incubated for 5 minutes at ambient temperature. To each tube was added 20 nM of arachidonic acid containing $10^6$ disintegrations per minute (DPM) of labelled arachidonic acid and the tubes incubated for 3 minutes at 37°C in a shaking water bath. After incubation the radioactive products were extracted from the acidified aqueous phase with ethyl acetate and after concentration resolved by thin layer chromatography on silica gel with chloroform/methanol/acetic acid/water (90:8:1:0.8) as a developing solvent. The amount of thromboxane produced was measured by scraping the radioactive zone corresponding to thromboxane $B_2$ and estimating the radioactivity in a liquid scintillation counter.

The concentration of active compound to reduce the enzyme activity by 50% ($ED_{50}$) was established. The results are shown in Table A.

The selectivity of the active compounds was measured in a similar manner to that described above and the amount of PGE, PGP and PGD produced was determined. The greater the selectivity, the more of the anti-aggregating prostaglandins are produced.

The $ED_{50}$ and Selectivity results are shown in Table A in which 0 indicates no selectivity; + low selectivity: ++ medium selectivity; +++ high selectivity, and ++++ exceptionally high selectivity.

TABLE A

| Compound (Reference Compound) | $ED_{50}$ $\mu$g/ml | Selectivity |
|---|---|---|
| (Imidazole) | $\gg$500 | 0 to + |
| (1-Methylimidazole) | $\gg$200 | ++ |
| 1-(3,4-dimethylbenzyl)imidazole | 6 | ++ |
| 1-[3-(2,4-dichlorophenyl)prop-2-enyl]imidazole | 4.1 | + |
| 1-[3-(2,6-dichlorophenyl)prop-2-enyl]imidazole | 1 | + |

Example 6

Tablet formulation

| | |
|---|---|
| 1-(3,4-dimethylbenzyl)imidazole (as a salt) | 150 mg |
| Starch | 25 mg |
| Polyvinylpyrrolidone | 2 mg |
| Magnesium stearate | 3 mg |

The imidazole salt is ground to a fine powder, blended with the starch and then the mixture granulated with an aqueous solution of the polyvinylpyrrolidones. The granules are sieved 1000 $\mu$, dried, sieved again and the magnesium stearate added. The mixture is then compressed into tablets.

In the same manner, tablets of 1-[3-(2,4-dichlorophenyl)prop-2-enyl]imidazole and 1 - [3 - (2,6 - dichlorophenyl)prop - 2 - enyl]imidazole are prepared.

Example 7

Tablet formulation

Tablets (150 mg) of the imidazoles described in the preceding example 6 are prepared in the same manner from the following ingredients:

| | |
|---|---|
| The Imidazole Compound (as a salt) | 150 mg |
| Lactose | 100 mg |
| Starch | 30 mg |
| Polyvinylpyrrolidone | 2 mg |
| Magnesium stearate | 3 mg |

In the preparation, the lactose is blended with the starch.

Example 8

Tablet formulation

Tablets (100 mg) of the imidazoles of Example 6 are prepared in the same manner from the following ingredients:

| | |
|---|---|
| The Imidazole Compound (as a salt) | 100 mg |
| Sodium starch glycollate | 10 mg |
| Polyvinylpyrrolidone | 2 mg |
| Magnesium stearate | 3 mg |

Example 9

Tablet formulation

Tablets (150 mg) of the imidazoles of Example 6 are prepared in the same manner from the following ingredients, except that the starch, pregelled starch and imidazole compound are all blended together prior to granulation:—

| | |
|---|---|
| The Imidazole Compound (as a salt) | 150 mg |
| Starch | 25 mg |
| Pregelled starch | 5 mg |
| Magnesium stearate | 3 mg |

### Example 10

Injectable formulation

| | |
|---|---|
| Imidazole compound of formula (I) | 15.0 g |
| Lactic Acid B.P. | q.s. to pH 3.0 |
| Water for Injections B.P. | to 1000 ml |

Suspend the compound in 3/4 of the available quantity of water. Add sufficient lactic acid to dissolve the compound and to reduce the pH to 3.0. Dilute to volume with Water for Injections.

Sterilise the solution by passage through a membrane filter, pore size 0.22 $\mu$m.

Distribute the solution using aseptic precautions into sterilised ampoules, 1 ml per ampoule. Seal by fusion of the glass.

Each 1 ml ampoule supplies 150 mg of the imidazole compound: 1-(3,4-dimethylbenzyl)imidazole hydrogen fumarate

### Example 11

Injectable formulation

| | |
|---|---|
| Imidazole compound of formula (I) | 15.0 g |
| Citric Acid B.P. | q.s. to pH 3.0 |
| Chlorocresol | 0.1 g |
| Water for Injections to | 100.0 ml |

Suspend the compound in $\frac{1}{2}$ the final volume of water for Injections. Add sufficient citric acid as a 10% solution in Water for Injections to dissolve the compound and reduce the pH to 3.0. Dilute to volume with Water for Injections.

Sterilise the solution by passage through a membrane filter, pore size 0.22 $\mu$m.

Distribute the solution with aseptic precautions into sterilised vials, 25 ml per vial. Stopper with sterile rubber closures and seal with an aluminium cap.

Each 1 ml of solution provides 150 mg of the compound: 1-(3,4-dimethylbenzyl)imidazole hydrogen fumarate.

### Example 12

Injectable formulation

In the manner described in the preceding two Examples, injectable formulations of 1 - [3 - (2,4 - dichlorophenyl)prop - 2 - enyl]imidazole and 1 - [3 - (2,6 - dichlorophenyl)prop - 2 - enyl]imidazole salts were prepared.

### Example 13

By the method described in Example 1 above the following compounds were prepared:—

(a) 1-(2,4,6-trimethylbenzyl)imidazole
(b) 1-[3-(3,4,5-trimethoxyphenyl)prop-2-enyl]imidazole
(c) 1-[3-(3,4-dimethoxyphenyl)prop-2-enyl]imidazole
(d) 1-[3-(2-hydroxyphenyl)prop-2-enyl]imidazole
(e) 1-[3-(3-bromophenyl)prop-2-enyl]imidazole
(f) 1-[3-(4-chlorophenyl)prop-2-enyl]imidazole
(g) 1-[3-(3,4-dimethylphenyl)prop-2-enyl]imidazole
(h) 1-[3-(2-methoxyphenyl)prop-2-enyl]imidazole

## Claims

1. A 1-arylalkylimidazole characterised in that said compound has the formula:

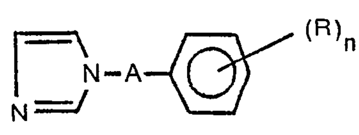

in which A is a straight or branched alkylene group of from 1 to 3 carbon atoms, or a straight or branched alkenylene or alkynylene group of 2 or 3 carbon atoms, n is an integer which is at least 1, and

the or each R substituent, which when n is greater than 1 may be the same or different, is a saturated alkyl group of from 1 to 4 carbon atoms or an unsaturated alkyl group of from 2 to 4 carbon atoms, with the provisos that

(a) when A is a methylene or ethylidene group, n is at least 2 when each R is saturated alkyl;

(b) when A is a branched propylene or straight propylidene group, n is at least 3 when each R is saturated alkyl;

(c) when A is unsaturated R may also be selected from alkoxy of from 1 to 4 carbon atoms; when n is at least 2, alkylenedioxy of from 1 to 4 carbon atoms; halo; trihalomethyl; hydroxy; carboxyl; a salt of such a carboxyl group; carboalkoxy; carboaryloxy; carboarylalkyloxy; $—NR^6R^7$ or $—CONR^6R^7$, in which $R^6$ and $R^7$ may be the same or different and are hydrogen or alkyl of from 1 to 4 carbon atoms; with the further proviso that when n is 1, R is not a saturated alkyl group;

or an acid addition salt of such a 1-arylalkylimidazole.

2. A 1-arylalkylimidazole as defined in claim 1 characterised in that A is $—CH_2—$ or, in the orientation of formula (I), $—CH_2—CH{=}CH—$.

3. A 1-arylalkylimidazole as defined in claim 2 characterised in that R and n together are 3,4-dimethyl substituents in the phenyl ring.

4. A 1-arylalkylimidazole characterised in that it is selected from 1 - (3,4 - dimethylbenzyl)imidazole, 1 - [3 - (2,4 - dichlorophenyl)prop - 2 - enyl]imidazole, and 1 - [3 - (2,6 - dichlorophenyl)prop - 2 - enyl]imidazole and acid addition salts thereof.

5. 1-(3,4-dimethylbenzyl)imidazole or an acid addition salt thereof.

6. 1-(3,4-dimethylbenzyl)imidazole.

7. A method of preparing a 1-arylalkylimidazole or an acid addition salt thereof as defined in claim 1 characterised in that one reacts imidazole or a salt thereof with an alkylating agent of the formula

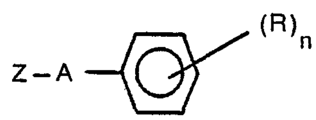

wherein A, n and R are as defined in claim 1 and Z is a leaving group.

8. A method of preparing a 1-arylalkylimidazole of the formula defined in any of claims 1 to 6 or an acid addition salt thereof characterised in that one

(a) converts a substituted imidazole of the formula:

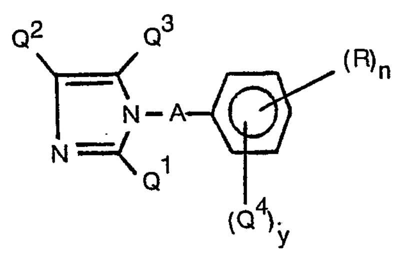

(IV)

wherein A, n and R are defined in claim 1 and $Q^1$, $Q^2$, $Q^3$ and $Q^4$ are the same or different, at least one being a radical capable of removal, the other(s) being a radical having the same or other removable function or is hydrogen, y is 0 or an integer, with the proviso that y and n together do not exceed 5;

(b) converts a precursor of the formula

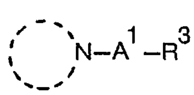

wherein

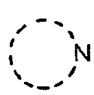

is 1-imidazoline, 1-imidazole or 1-pyrazole, $A^1$ is a straight or branched, saturated or unsaturated acyclic hydrocarbon radical which may include a keto group, and $R^3$ is

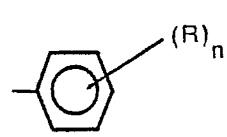

as defined in claim 1 and R may also be nitro if A is unsaturated, provided that at least one of

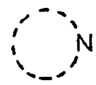

$A^1$ and $R^3$ is other than 1-imidazole, a saturated acyclic hydrocarbon group and

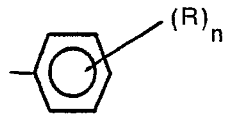

as defined in claim 1;

(c) alkylates a compound of formula (X):

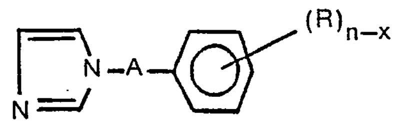

(X)

wherein A, R and n are as defined in claim 1 and x is an integer less than or equal to n with a compound of formula $RZ^1$ wherein R is an alkyl group as defined in claim 1 and $Z^1$ is a leaving group

(d) cyclises a compound of formula (XI):

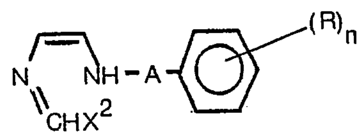

(XI)

wherein A, R and n are as defined for claim 1 and $X^2$ is a leaving group;

(e) reacts a compound of formula (XII):

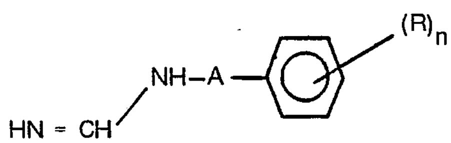

(XII)

wherein A, R and n are as defined in claim 1 (I) with a compound of formula (XIII):

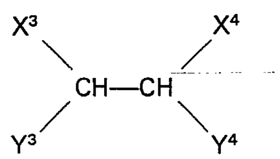

(XIII)

wherein either of $X^3$ and $Y^3$ is a leaving group and the other is hydrogen or $X^3$ and $Y^3$ are both halo or together form a keto group or an acetal derivative thereof, and $X^4$ and $Y^4$ are as defined for $X^3$ and $Y^3$, being the same as or different from $X^3$ and $Y^3$; or

(f) reduces an imine salt of formula (XIIIa):

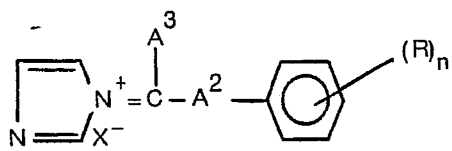

(XIIIa)

wherein R and n are as for formula (I), $X^-$ is an anion, $A^2$ is a chemical bond or a straight or branching, saturated or unsaturated acyclic hydrocarbon radical, which may include a keto group, $A^3$ is hydrogen or a saturated or unsaturated acyclic hydrocarbon radical, which may include a keto group, with the proviso that $A^2$ and $A^3$ together contain no more than 2 carbon atoms.

9. A pharmaceutical formulation of a 1-aryl-alkylimidazole characterised in that it comprises an imidazole according to any one of claims 1 to 6 or prepared by a process according to claim 7 or 8 or a

pharmaceutically acceptable salt thereof, together with a pharmaceutically acceptable carrier therefor.

10. A pharmaceutical formulation as claimed in claim 9 characterised in that it is in the form of a tablet.

11. A pharmaceutical formulation as claimed in claim 9 characterised in that it is in the form of a parenterally acceptable injectable solution or suspension.

12. A pharmaceutical formulation as claimed in claim 9 characterised in that it is in the form of a capsule

13. A tablet of 1-(3,4-dimethylbenzyl)imidazole or a pharmaceutically acceptable salt thereof.

14. A tablet as claimed in claim 13 characterised in that it contains from 50 to 500 mg of the imidazole or a pharmaceutically acceptable salt thereof, said amount being the amount of the imidazole base present.

15. A method of preparing a pharmaceutical formulation characterised in that one admixes a 1-arylalkylimidazole or a pharmaceutically acceptable acid addition salt thereof as defined in claim 1 with a pharmaceutically acceptable carrier thereof.

16. A 1-arylalkylimidazole as defined in any one of claims 1 to 6 or prepared by a process according to claim 7 or 8 or a pharmaceutically acceptable salt thereof, as an active agent for the treatment or prophylaxis of thrombo-embolic disorders of a mammal or mammalian tissue.

17. 1-(3,4-dimethylbenzyl)imidazole or a pharmaceutically acceptable acid addition salt thereof as an active agent for the treatment or prophylaxis of thrombi in a mammal or mammalian tissue.

18. 1-(3,4-dimethylbenzyl)imidazole or a pharmaceutically acceptable acid addition salt thereof as an active agent for the treatment or prophylaxis of myocardial infarction.

19. A pharmaceutical formulation according to any one of claims 9 to 14 as an active agent for the treatment or prophylaxis of a thrombo-embolic disorder in a mammal or mammalian tissue.

**Revendications**

1. 1-Arylalcoylimidazole, caractérisé en ce que ce composé répond à la formule:

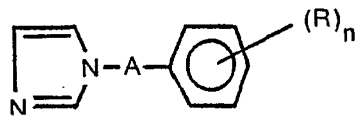

où A est un radical alcoylène en chaîne droite ou ramifiée de 1 à 3 atomes de carbone ou un radical acénylène ou alcynylène en chaîne droite ou ramifiée de 2 ou 3 atomes de carbone

n est un nombre entier valant au moins 1 et

le ou chaque substituant R, qui lorsque R est supérieur à 1, peut être identique ou différent, est un radical alcoyle saturé de 1 à 4 atomes de carbone ou un radical alcoyle insaturé de 2 à 4 atomes de carbone, avec les restrictions

(a) que lorsque A est un radical méthylène ou éthylidène, n vaut au moins 2 lorsque chaque R est un radical alcoyle saturé;

(b) que lorsque A est un radical propylène ramifié ou propylidène en chaîne droite, n vaut au moins 3 lorsque chaque R est un radical alcoyle saturé;

(c) que lorsque A est insaturé, R peut aussi être choisi parmi les radicaux alcoxy de 1 à 4 atomes de carbone lorsque n vaut au moins 2, les radicaux alcoylènedioxy de 1 à 4 atomes de carbone, les radicaux halogéno, le radical trihalogénométhyle, le radical hydroxyle, le radical carboxyle, un sel d'un tel radical carboxyle, les radicaux carboalcoxy, les radicaux carboaryloxy, les radicaux carboarylalcoyloxy, les radicaux $-NR^6R^7$ ou $-CONR^6R^7$ où $R^6$ et $R^7$ qui peuvent être identiques ou différents sont des atomes d'hydrogène ou radicaux alcoyle de 1 à 4 atomes de carbone, avec la restriction supplémentaire que lorsque n vaut, R n'est pas un radical alcoyle saturé;

ou un sel d'addition d'acide d'un tel 1-arylalcoylimidazole.

2. 1-Arylalcoylimidazole suivant la revendication 1, caractérisé en ce que A est un radical $-CH_2-$ ou dans l'orientation de la formule (I), $-CH_2-CH{=}CH-$,

3. 1-Arylalcoylimidazole suivant la revendication 2, caractérisé en ce que R et n représentent ensemble des radicaux 3,4-diméthyle sur le cycle phényle.

4. 1-Arylalcoylimidazole, caractérisé en ce qu'il est choisit entre le 1-(3,4-diméthylbenzyl)imidazole, le 1 - [3 - (2,4 - dichlorophényl)prop - 2 - ényl]imidazole et le 1 - [3 - (2,6 - dichlorophényl)prop - 2 - ényl]imidazole et leurs sels d'addition d'acides.

5. Le 1-(3,4-diméthylbenzyl)imidazole ou un sel d'addition d'acide de celui-ci.

6. Le 1-(3,4-diméthylbenzyl)imidazole.

7. Procédé de préparation d'un 1-arylalcoylimidazole ou d'un sel d'addition d'acide de celui-ci tel que défini dans la revendication 1, caractérisé en ce qu'on fait réagir l'imidazole ou un sel de celui-ci avec un agent d'alcoylation de formule

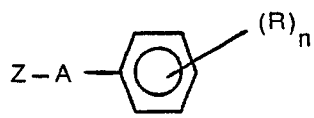

où A, n et R sont tels que définis dans la revendication 1 et Z est un radical labile.

8. Procédé de préparation d'un 1-arylalcoylimidazole de la formule définie dans l'une quelconque des revendications 1 à 6 ou d'un sel d'addition d'acide de celui-ci, caractérisé en ce que

(a) on convertit un imidazole substitué de formule

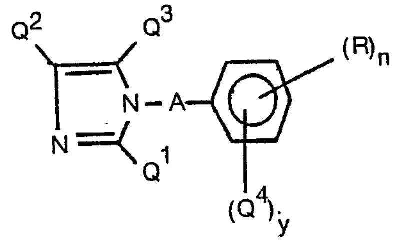 (IV)

où A, n et R sont tels que définis dans la revendication 1 et $Q^1$, $Q^2$, $Q^3$ et $Q^4$ sont identiques ou différents, au moins l'un d'entre eux étant un radical capable d'élimination, le ou les autres étant des radicaux ayant la même fonction éliminable ou une autre ou étant un atome d'hydrogène, y est 0 ou un nombre entier, avec la restriction que y et n ensemble n'excèdent pas 5;

(b) on convertit un précurseur de formule

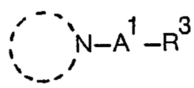 (VII)

ou

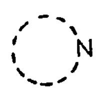

est la 1-imidazoline, le 1-imidazole ou le 1-pyrazole, $A^1$ est un radical hydrocarboné acyclique saturé ou insaturé en chaîne droite ou ramifiée qui peut comprendre un radical céto et $R^3$ est un radical

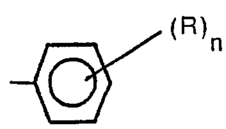

tel que défini dans la revendication 1 et R peut aussi être un radical nitro si A est insaturé, avec la restriction qu'au moins l'un d'entre

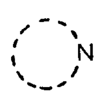

$A^1$ et $R^3$ est autre que le 1-imidazole un radical hydrocarboné acyclique saturé et

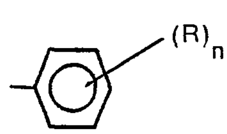

comme défini dans la revendication 1.

(c) on opère l'alcoylation d'un composé de formule (X):

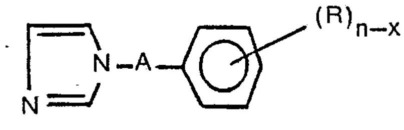 (X)

où A, R et n sont tels que définis dans la revendication 1 et x est un nombre entier inférieur ou égal à n au moyen d'un composé de formule $RZ^1$ où R est un radical alcoyle tel que défini dans la revendication 1 et $Z^1$ est un radical labile;

(d) on cyclise un composé de formule (XI):

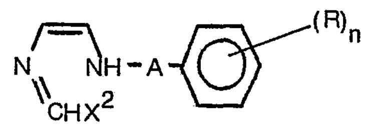

(XI)

où A, R et n sont tels que définis dans la revendication 1 et $X^2$ est un radical labile;

(e) on fait réagir un composé de formule (XII):

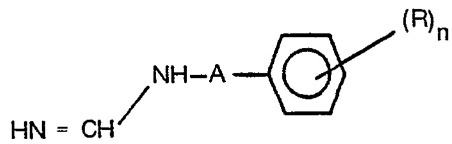

(XII)

où A, R et n sont tels que définis dans la revendication 1 avec un composé de formule (XIII):

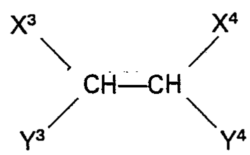

(XIII)

où l'un d'entre $X^3$ et $Y^3$ est un radical labile et l'autre est un atome d'hydrogène ou bien $X^3$ et $Y^3$ sont tous deux des radicaux halogéno ou forment ensemble un radical céto ou un acétal qui dérive de celui-ci et $X^4$ et $Y^4$ sont définis comme $X^3$ et $Y^3$, étant identiques à $X^3$ et $Y^3$ ou différents de ceux-ci, ou

(f) on réduit un sel d'imine de formule (XIIIa)

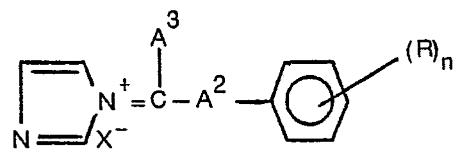

(XIIIa)

où R et n sont tels que définis dans la formule (I), $X^-$ est un anion, $A^2$ est un liaison chimique ou un radical hydrocarboné acyclique saturé ou insaturé en chaîne droite ou ramifiée qui peut comprendre un radical céto, $A^3$ est un atome d'hydrogène ou un radical hydrocarboné acyclique saturé ou insaturé qui peut comprendre un radical céto, avec la restriction que $A^2$ et $A^3$ ne contiennent ensemble pas plus de 2 atomes de carbone.

9. Composition pharmaceutique d'un 1-arylalcoylimidazole, caractérisé en ce qu'elle comprend un imidazole suivant l'une quelconque des revendications 1 à 6 ou préparé par un procédé suivant la revendication 7 ou 8 ou un sel pharmaceutiquement acceptable de ce composé, outre un excipient pharmaceutiquement acceptable.

10. Composition pharmaceutique suivant la revendication 9, caractérisée en ce qu'elle se présente sous forme d'une tablette.

11. Composition pharmaceutique suivant la revendication 9, caractérisée en ce qu'elle se présente sous la forme d'une solution ou suspension à injecter parentéralement acceptable.

12. Composition pharmaceutique suivant la revendication 9, caractérisée en ce qu'elle se présente sous la forme d'une capsule.

13. Tablette de 1-(3,4-diméthylbenzyl)imidazole ou d'un sel pharmaceutiquement acceptable de celui-ci.

14. Tablette suivant la revendication 13, caractérisée en ce qu'elle contient 50 à 500 mg de l'imidazole ou d'un sel pharmaceutiquement acceptable de celui-ci, cette quantité étant la quantité d'imidazole base en présence.

15. Procédé de préparation d'une composition pharmaceutique, caractérisé en ce qu'on mélange un 1-arylalcoylimidazole ou un sel pharmaceutiquement acceptable de celui-ci tel que défini dans la revendication 1 avec un excipient pharmaceutiquement acceptable.

16. 1-Arylalcoylimidazole tel que défini dans l'une quelconque des revendications 1 à 6 ou préparé par un procédé suivant la revendication 7 ou 8 ou un sel pharmaceutiquement acceptable de celui-ci comme agent actif pour le traitement ou la prophylaxie des troubles thrombo-emboliques d'un

mammifère ou tissu de mammifère.

17. Le 1-(3,4-diméthylbenzyl)imidazole ou un sel d'addition d'acide pharmaceutiquement acceptable de celui-ci comme agent actif pour le traitement ou la prophylaxie d'un thrombus d'un mammifère ou tissu de mammifère.

18. Le 1-(3,4-diméthylbenzyl)imidazole ou un sel d'addition d'acide pharmaceutiquement acceptable de celui-ci comme agent actif pour le traitement ou la prophylaxie de l'infarctus du myocarde.

19. Composition pharmaceutique suivant l'une quelconque des revendications 9 à 14 comme agent actif pour le traitement ou la prophylaxie d'un trouble thromboembolique d'un mammifère ou d'un tissu de mammifère.

**Patentansprüche**

1. 1-Arylalkylimidazole, gekennzeichnet durch die allgemeine Formel I

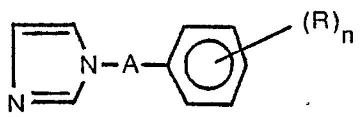

(I)

worin A einen geradkettigen oder verzweigten Alkylenrest mit 1 bis 3 Kohlenstoffatomen oder einen geradkettigen oder verzweigten Alkenylen- oder Alkinylenrest mit 2 bis 3 Kohlenstoffatomen bedeutet, n eine ganze Zahl von mindestens 1 darstellt, un der oder jeder Substituent R, der bei einem Wert von n größer als 1 gleich oder unterschiedlich sein kann, einen gesättigten Alkylrest mit 1 bis 4 Kohlenstoffatomen oder einen ungesättigten Alkylrest mit 2 bis 4 Kohlenstoffatomen darstellt, mit den Maßgaben, daß

(a) wenn A eine Methylen- oder Ethylidengruppe ist, n mindestens 2 ist, sofern jedes R einen gesättigten Alkylrest darstellt,

(b) wenn A eine verzweigte Propylengruppe oder eine geradkettige Propylidengruppe bedeutet, n mindestens 3 ist, sofern jedes R einen gesättigten Alkylrest darstellt,

(c) wenn A ungesättigt ist, R auch einen Alkoxyrest mit 1 bis 4 Kohlenstoffatomen bedeuten kann; wenn n mindestens 2 ist, Alkylendioxy mit 1 bis 4 Kohlenstoffatomen; Halogen; Trihalogenmethyl; Hydroxy; Carboxyl; ein Salz eines derartigen Carboxylrestes; Carboalkoxy; Carboaryloxy; Carboarylalkyloxy; $—NR^6R^7$ oder $—CONR^6R^7$, worin $R^6$ und $R^7$ gleich oder unterschiedlich sein können und Wasserstoff oder Alkyl mit 1 bis 4 Kohlenstoffatomen darstellen; mit der weiteren Maßgabe, daß bei einem Wert von n gleich 1, R keinen gesättigten Alkylrest bedeutet; oder ein Säureadditionssalz derartiger 1-Arylalkylimidazole.

2. 1-Arylalkylimidazol nach Anspruch 1, dadurch gekennzeichnet, daß A gleich $—CH_2—$ ist oder in der Orientierung von Formel I gleich $—CH_2—CH{=}CH—$.

3. 1-Arylalkylimidazol nach Anspruch 2, dadurch gekennzeichnet, daß R und n zusammen 3,4-Dimethylsubstituenten in dem Phenylring sind.

4. 1-Arylalkylimidazol, dadurch gekennzeichnet, daß es unter 1-(3,4-Dimethylbenzyl)imidazol, 1 - [3 - 2,4 - Dichlorphenyl)prop - 2 - enyl]imidazol und 1 - [3 - (2,6 - Dichlorphenyl)prop - 2 - enyl]imidazol und deren Säureadditionssalzen ausgewählt ist.

5. 1-(3,4-Dimethylbenzyl)imidazol oder eines seiner Säureadditionssalze.

6. 1-(3,4-Dimethylbenzyl)imidazol.

7. Verfahren zur Herstellung von 1-Arylalkylimidazolen oder deren Säureadditionssalzen nach Anspruch 1, dadurch gekennzeichnet, daß ein Imidazol oder ein entsprechendes Salz mit einem Alkylierungsmittel der Formel

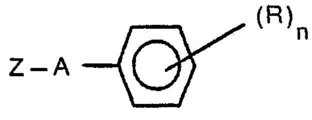

worin A, n und R die in Anspruch 1 genannte Bedeutung haben und Z eine Abspaltgruppe ist, umgesetzt wird.

8. Verfahren zur Herstellung von 1-Arylalkylimidazolen nach der Formel gemäß der Definition in einem der Ansprüche 1 bis 6 oder eines entsprechenden Säureadditionssalzes, dadurch gekennzeichnet, daß

(a) eine substituiertes Imidazol der Formel

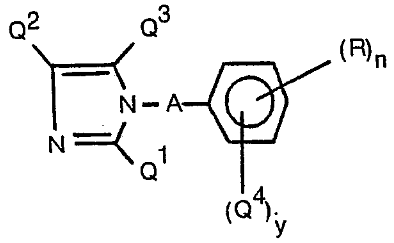

(IV)

worin A, n und R die in Anspruch 1 genannte Bedeutung haben und $Q^1$, $Q^2$, $Q^3$ und $Q^4$ gleich oder unterschiedlich sind, wobei mindestens eines ein abspaltbarer Rest ist, und die anderen jeweils Reste sind, welche die gleiche oder andere abtrennbare Funktionen aufweisen oder Wasserstoff sind, Y gleich 0 oder eine ganze Zahl ist, mit der Maßgabe, daß Y und n zusammen den Wert 5 nicht überschreiten, umgewandelt wird,

(b) ein Vorstufe der Formel

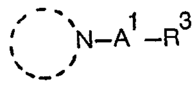

(VII)

worin

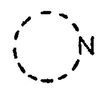

1-Imidazolin, 1-Imidazol oder 1-Pyrazol bedeutet, $A^1$ einen geradkettigen oder verzweigten gesättigten oder ungesättigten acyclischen Kohlenwasserstoffrest bedutet, der einen Ketorest umfassen kann, und $R^3$ für

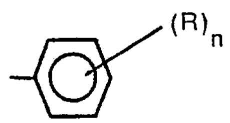

gemäß der Definition in Anspruch 1 steht und R auch Nitro sein kann, sofern A ungesättigt ist, mit der Maßgabe, daß mindestens einer der Reste

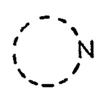

$A^1$ und $R^3$ etwas anderes darstellt als 1-Imidazol, einen gesättigten acyclischen Kohlenwasserstoffrest und

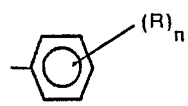

gemäß der Definition in Anspruch 1, überführt wird,

(c) eine Verbindung der allgemeinen Formel X

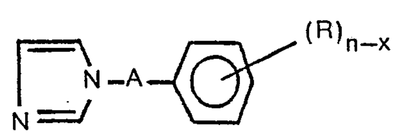

(X)

worin A, R und n die in Anspruch 1 genannte Bedeutung haben und x eine ganze Zahl von weniger oder gleich n ist, mit einer Verbindung der Formel $RZ^1$, in der R einen Alkylrest gemäß der Definition in Anspruch 1 darstellt, und $Z^1$ eine Abspaltgruppe ist, alkyliert wird.

(d) eine Verbindung der allgemeinen Formel XI

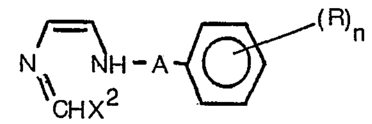

(XI)

worin A, R und n die in Anspruch 1 genannte Bedeutung haben und $X^2$ eine Abspaltgruppe ist, cyclisiert wird,

(e) eine Verbindung der allgemeinen Formel XII

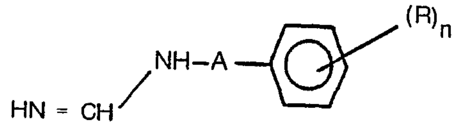

(XII)

worin A, R und n die in Anspruch 1 genannte Bedeutung haben, mit einer Verbindung der allgemeinen Formel XIII

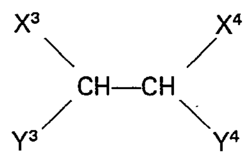

(XIII)

worin entweder $X^3$ oder $Y^3$ ein Abspaltgruppe darstellt und der andere Rest Wasserstoff ist oder $X^3$ und $Y^3$ beide Halogenatome sind oder zusammen eine Ketogruppe bilden oder ein entsprechendes Acetalderivat, und $X^4$ und $Y^4$ der Definition für $X^3$ und $Y^3$ entsprechen, wobei sie gleich $X^3$ und $Y^3$ sind oder von $X^3$ und $Y^3$ unterschiedlich sind, umgesetzt wird, oder

(f) ein Iminsalz der allgemeinen Formel XIIIa

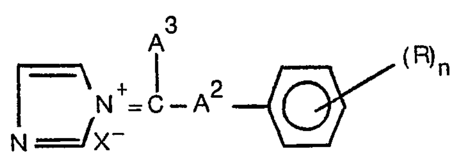

(XIIIa)

worin R und n der Definition zu Formel I entsprechen, $X^-$ ein Anion ist, $A^2$ eine chemische Bindung oder einen geradkettigen oder verzweigten, gesättigten oder ungesättigten acyclischen Kohlenwasserstoffrest, der eine Ketogruppe umfassen kann, darstellt, $A^3$ ein Wasserstoffatom oder einen gesättigten oder ungesättigten acyclischen Kohlenwasserstoffrest, der eine Ketogruppe umfassen kann, darstellt, mit der Maßgabe, daß $A^2$ und $A^3$ zusammen nicht mehr als zwei Kohlenstoffatome enthalten, reduziert wird.

9. Pharmazeutische Zusammensetzung eines 1-Arylalkylimidazols, dadurch gekennzeichnet, daß sie ein Imidazol nach einem der Ansprüche 1 bis 6 oder ein durch ein Verfahren nach einem der Ansprüche 7 und 8 hergestelltes 1-Arylalkylimidazol oder ein entsprechendes pharmazeutisch verträgliches Salz, zusammen mit einem pharmazeutisch verträglichen Träger dafür enthält.

10. Pharmazeutische Formulierung nach Anspruch 9, dadurch gekennzeichnet, daß sie in Form einer Tablette vorliegt.

11. Pharmazeutische Formulierung nach Anspruch 9, dadurch gekennzeichnet, daß sie in Form einer parenteral verträglichen injizierbaren Lösung oder Suspension vorliegt.

12. Pharmazeutische Formulierung nach Anspruch 9, dadurch gekennzeichnet, daß sie in Form einer Kapsel vorliegt.

13. Tablette des 1-(3,4-Dimethylbenzyl)imidazols oder eines pharmazeutisch verträglichen Salzes davon.

14. Tablette nach Anspruch 13, dadurch gekennzeichnet, daß sie 50 bis 500 mg des Imidazols oder eines pharmazeutisch verträglichen Salzes davon enthält, wobei die Menge die Menge der im Imidazol vorhandenen Base ist.

15. Verfahren zur Herstellung einer pharmazeutischen Formulierung, dadurch gekennzeichnet, daß ein 1-Arylalkylimidazol oder ein entsprechendes pharmazeutisch verträgliches Salz davon gemäß der Definition in Anspruch 1 mit einem pharmazeutisch verträglichen Träger dafür vermischt wird.

16. 1-Arylalkylimidazol nach einem der Ansprüche 1 bis 6 oder hergestellt nach einem Verfahren

gemäß Anspruch 7 oder 8 oder ein pharmazeutisch verträgliches Salz davon, als ein wirksames Mittel zur Behandlung oder Prophylaxe von thrombo-embolischen Erkrankungen eines Säugetiers oder Säugetiergewebes.

17. 1-(3,4-Dimethylbenzyl)imidazol oder ein pharmazeutisch verträgliches Säureadditionssalz davon als ein aktives Mittel zur Behandlung oder Prophylaxe von Thromben in einem Säugetier oder Säugetiergewebe.

18. 1-(3,4-Dimethylbenzyl)imidazol oder ein pharmazeutisch verträgliches Säureadditionssalz davon als ein aktives Mittel zur Behandlung oder Prophylaxe von Myocardialinfarkt.

19. Pharmazeutische Formulierung nach einem der Ansprüche 9 bis 14 als ein aktives Mittel zur Behandlung oder Prophylaxe von thrombo-embolischen Erkrankungen eines Säugetiers oder von Säugetiergewebe.